# EUROPÄISCHE PATENTSCHRIFT

(11) **EP 1 845 876 B1**
(45) Veröffentlichungstag und Bekanntmachung des Hinweises auf die Patenterteilung: **20.07.2011**
(21) Anmeldenummer: 06705937.8
(22) Anmeldetag: 08.02.2006
(51) Int. Cl.: A61B 17/70, A61B 17/02

(54) **DORNFORTSATZSPREIZER**
DEVICE FOR FORCING APART SPINOUS PROCESSES
DISPOSITIF POUR ELARGIR DES APOPHYSES EPINEUSES

(30) Priorität: 08.02.2005 DE 102005005694; 16.03.2005 US 661926 P
(43) Veröffentlichungstag der Anmeldung: 24.10.2007
(73) Patentinhaber: Kloss, Henning, 6373 Ennetbürgen (CH)
(72) Erfinder: SCHÄFER, Björn, 53809 Ruppichteroth (DE)
(74) Vertreter: Arth, Hans-Lothar
(86) Internationale Anmeldenummer: PCT/DE2006/000212
(87) Internationale Veröffentlichungsnummer: WO 2006/084444

(56) Entgegenhaltungen:
- WO-A-02/07623
- WO-A-03/007791
- US-A1- 2002 143 331
- US-A1- 2004 153 065
- US-A1- 2005 165 398

## Beschreibung

Die vorliegende Erfindung betrifft eine Vorrichtung zum Spreizen der Dornfortsätze zweier aufeinanderfolgender Wirbel. Dabei liegen die Dornfortsätze auf stufenlos aufweitbaren Spreizmitteln. Beide Spreizmittel sind an einem Grundkörper angebracht, der stufenlos aufgeweitet werden kann und wobei das Ausmaß der Aufweitung den Abstand der Spreizmittel und dadurch den Grad der Spreizung der Dornfortsätze zueinander bestimmt.

Im Stand der Technik sind nur Ausführungsformen zum Spreizen von Domfortsätzen bekannt, welche keine linear stufenlose Aufweitung bzw. Spreizung der Dornfortsätze zweier benachbarter Wirbel zulassen. Die Spreizung wird durch Abstandshalter definierter Höhe bewerkstelligt.

So offenbart US 2003/0065330 A1 eine Vorrichtung zum Spreizen der Dornfortsätze von Wirbeln, wobei auf einer horizontalen Schiene zwei vertikale Fixierungsmittel angebracht sind. Die Dornfortsätze der Wirbel liegen auf einem über die horizontale Schiene aufschiebbaren Abstandshalter auf, dessen Dicke das Maß der Aufspreizung bestimmt. Nach erfolgter Implantation ist der Grad der Aufspreizung nicht mehr veränderbar.

Eine Vorrichtung zur zwar stufenlosen, aber nicht linear stufenlosen Aufweitung offenbart das US-Patent US 6,733,534 B2. Darin wird ein befüllbarer elastischer Hohlkörper aus einem polymeren Material beschrieben, der zwischen zwei benachbarte Wirbel platziert wird und danach durch Befüllung mit einem biologischen Material bis zu einer bestimmten Dicke aufgeweitet wird.

-WO 02/07623 offenbart eine Vorrichtung zum Spreizen der Dornfortsätze von Wirbeln gemäß dem Oberbegriff des Anspruche 1.-Aufgabe der vorliegenden Erfindung ist die Bereitstellung einer Vorrichtung zum Spreizen der Dornfortsätze zweier aufeinanderfolgender Wirbel, welche es erlaubt, den Grad der Spreizung linear stufenlos während der Implantation einzustellen.

Diese Aufgabe wird durch die Bereitstellung einer Vorrichtung gemäß Patentanspruch 1 gelöst. Weitere vorteilhafte Ausgestaltungen, Aspekte und Details der Erfindung ergeben sich aus den abhängigen Ansprüchen, der Beschreibung, den Beispielen und den Figuren.

Die vorliegende Erfindung betrifft eine Vorrichtung zum Spreizen der Dornfortsätze zweier aufeinanderfolgender Wirbel, umfassend einen Grundkörper 1 mit mindestens zwei Spreizmitteln 2A und 2B, wobei die mindestens zwei Spreizmittel 2 stufenlos aufgeweitet werden können. Vorzugsweise sind zwei oder vier Spreizmittel vorhanden, also ein oder zwei Spreizmittel pro Dornfortsatz. Insbesondere bevorzugt sind zwei Spreizmittel 2A und 2B, also nur ein Spreizmittel pro Domfortsatz.

Als Spreizmittel 2 wird der Teil der Vorrichtung bezeichnet, auf den die Dornfortsätze hauptsächlich aufliegen. Erfindungsgemäß sind mindestens zwei Spreizmittel 2 erforderlich, nämlich pro Dornfortsatz ein Spreizmittel 2, auf das der jeweilige Dornfortsatz aufliegt. Durch Aufweitung der beiden Spreizmittel 2A, 2B, d.h. durch Bewegung der beiden Spreizmittel 2A und 2B entlang der axialen Achse voneinander weg, erfolgt die Spreizung der Dornfortsätze und damit der beiden zu behandelnden Wirbel. Beide Spreizmittel sind also erfindungsgemäß entlang der axialen Achse, d.h. entlang der Längsachse durch die Wirbelsäule linear stufenlos aufweitbar. Die Aufweitung des Implantats erfolgt nur entlang der axialen Achse. Das Implantat verändert in lateraler Richtung als auch in ventraler und dorsaler Richtung seine Größe nicht. Als ventrale-dorsale Achse wird diejenige bezeichnet, welche durch den Rücken und Bauch verläuft. Dazu senkrecht verläuft die axiale Achse entlang der Wirbelsäule und wiederum senkrecht zur axialen Achse verläuft die laterale Achse seitlich durch den Körper.

Die Distraktion der Spreizmittel erfolgt also nur entlang der Achse durch die Wirbelsäule stufenlos, so dass das implantat auch nur in Richtung dieser Achse seine Größe verändert. Die Aufweitung der Spreizmittel erfolgt somit entlang einer Geraden und nicht entlang einer Kurvenlinie. Zudem weist das erfindungsgemäße Implantat keine flexiblen Komponenten auf, welche eine gewisse Rückstellung aufgrund des Druckes der Dornfortsätze zeigen oder dem Druck der Dornfortsätze nachgeben und die Distraktionsstrecke damit wieder verkleinern.

Unter linearer Aufweitung soll verstanden werden, dass eine bestimmte Strecke, welche ein externes oder implantateigenes Mittel zur Aufweitung der Spreizmittel zurücklegt, zu einer definierten Aufweitung der Spreizmittel führt. Wird beispielsweise ein externes Mittel zum Aufweiten der Spreizmittel verwendet, so führt beispielsweise eine Bewegung dieses Mittels von 1 mm zu einer Aufweitung der Spreizmittel von z.B. 1 mm. Jede weitere Bewegung dieses Mittels führt weiterhin immer zu einer weiteren Aufweitung der Spreizmittel von 1 mm. Wird beispielsweise das hierin beschriebene Fixierungsmittel mit kegelförmiger Spitze zur Aufweitung der Spreizmittel eingesetzt, so führt beispielsweise eine 360° Drehung des Fixierungsmittels zu einer Aufweitung der Spreizmittel von 1,8 mm. Jede weitere 360° Drehung bewirkt weitere Aufweitungen von jeweils 1,8 mm. Eine derartige Aufweitung wird als stufenlose lineare Aufweitung bezeichnet.

Im Gegensatz dazu führt das in US 6,733,534 B2 beschriebene Verfahren zur Aufweitung auch zu einer stufenlosen, aber nicht linearen Aufweitung, da beispielsweise die Befüllung mit einem ersten ml an biologischem Material zu einer Aufweitung von 4 mm, aber die Befüllung mit einem vierten ml biologischem Materials z.B. nur noch zu einer Aufweitung von 1,2 mm führt. Nachteilig bei einer nicht linearen Aufweitung ist, dass der Operateur den erforderlichen Druck nur ungenau einstellen kann. Zudem ist bei elastischen implantaten, wie z.B. in US 6,733,534 B2 beschrieben, ein Problem, dass sich das Implantat nach der Implantation aufgrund der Elastizität verformen kann und die gewünschte optimale Aufspreizung wieder verloren geht.

Erfindungsgemäß erfolgt also die Aufspreizung, d.h. die Bewegung der beiden Spreizmittel 2A und 2B entlang der axialen Achse linear und stufenlos. Es werden nicht vorgefertigte Abstandshalter mit vorbestimmter Höhe zwischen den Spreizmitteln platziert, sondern die Spreizmittel 2A und 2B können zwischen einem Mindestabstand und einem Höchstabstand zueinander, linear und stufenlos jeden beliebigen Abstand zueinander einnehmen. Der Mindestabstand wird durch den Durchmesser beider fest aufeinanderliegender Spreizmittel 2A und 2B bestimmt und liegt vorzugsweise bei ca. 5 mm. Der stufenlos einstellbare Bereich der erfindungsgemäßen Vorrichtung, d.h. der Abstand zwischen den beiden Spreizmitteln 2A und 2B beträgt 5-25 mm, bevorzugt 6,5 - 20 mm und insbesondere bevorzugt 8-16 mm.

Das stufenlose Bewegen der beiden Spreizmittel 2A und 2B entlang der axialen Achse zueinander wird erfindungsgemäß dadurch erreicht, dass jedes Spreizmittel 2 auf einem Führungsmittel 3 befestigt ist und die Führungsmittel 3 durch einen Grundkörper 1 fixiert, jedoch in axialer Richtung beweglich gelagert sind. Dabei können auch beide Spreizmittel auf ein und demselben Führungsmittel 3 angeordnet sein.

Nach Distraktion und Fixierung der Spreizmittel bleibt die gewählte Distraktionsstrecke unverändert. Das Implantat verändert seine Größe in axialer Richtung nach endgültiger Fixierung nicht mehr, insbesondere nicht mehr von alleine.

In der bevorzugten Ausführungsform mit zwei Spreizmitteln 2A und 2B ist das Spreizmittel 2A an dem Führungsmittel 3A und das Spreizmittel 2B an dem Führungsmittel 3B angebracht. Beide Führungsmittel 3A und 3B sind an einem Grundkörper 1 oder in einem Grundkörper derart gelagert, das zumindest ein Führungsmittel 3A oder 3B und vorzugsweise beide Führungsmittel 3A und 3B relativ zueinander in Richtung der axialen Achse verschoben oder distrahiert bzw. auseinandergeschoben oder auseinanderbewegt werden können.

Als Führungsmittel 3A und 3B können beispielsweise Führungsschienen; Führungsstäbe oder Führungsrohre verwendet werden, welche im oder am Grundköper 1 in entsprechenden Aussparungen oder Aufnahmemitteln entlang der axialen Achse beweglich gelagert sind. Durch ein Verschieben der Führungsmittel 3 kann ein beliebiger Abstand zischen den Spreizmitteln 2A und 2B eingestellt werden. Durch entsprechende Fixierungsmittel 4 werden dann die Führungsmittel 3 in ihrer Position fixiert, wodurch garantiert wird, das der Abstand zwischen den Spreizmitteln 2A und 2B dauerhaft erhalten bleibt und der eingestellte Abstand, d.h. der Grad der Spreizung der Wirbel nicht durch den auf die Spreizmittel wirkenden Druck der Wirbelfortsätze wieder verringert wird.

Bei einer bevorzugten Ausführungsform werden nicht die Spreizmittel 2A und 2B auf beweglichen Führungsmitteln 3A und 3B befestigt, welche wiederum an einem einteiligen Grundkörper 1 beweglich angebracht sind, sondern es werden zweiteilige, dreiteilige oder mehrteilige Grundkörper eingesetzt, welche nur entlang der axialen Achse aufweitbar, dehnbar und/oder auseinanderschiebbar sind.

Insbesondere bevorzugt sind zweiteilige Grundkörper 1, wobei an dem einen Teil des Grundkörpers 1A das Spreizmittel 2A und an dem anderen Teil des Grundkörpers 1 B das Spreizmittel 2B translationsstabil befestigt ist. Unter translationsstabiler Befestigung wird eine ortsfeste Anbringung verstanden, welche eine Rotationsbewegung des Spreizmittels zulassen kann, jedoch keine Translationsbewegung von Spreizmittel 2A (bzw. 2B) relativ zum Teil 1A (bzw. 1B) des Grundkörpers erlaubt. Die Spreizmittel 2A und 2B sind vorzugsweise auf derselben Seite des jeweiligen Grundkörpers angebracht und weniger bevorzugt auf gegenüberliegenden Seiten.

Bei diesen bevorzugten Ausführungsformen sind nicht die Spreizmittel 2 beweglich auf dem Grundkörper 1 angebracht, sondern sind unverschiebbar auf einem Teil 1A bzw. 1B des Grundkörpers befestigt und werden durch das Auseinanderschieben der beiden Teile 1A und 1B des Grundkörpers 1 auseinander bewegt.

Der vorzugsweise zweiteilige oder mehrteilige Grundkörper 1 besteht somit aus zwei oder mehreren ineinandergreifenden, ausziehbaren, distrahierbaren, aufweitbaren, ineinander schiebbaren und/oder aneinander entlang gleitbaren Teilen. Insbesondere bevorzugt sind zweiteilige Grundkörper 1 aus den beiden Teilen 1 A und 1B. Die beiden Spreizmittel 2A und 2B befinden sich an zwei unterschiedlichen Teilen des Grundkörpers und vorzugsweise auf derselben Seite des Grundkörpers.

Mindestens ein Teil 1A oder 1B des Grundkörpers 1 ist mit mindestens einem Führungsmittel 3 ausgestattet. Wird in einer bevorzugten Ausführungsform nur ein Führungsmittel 3 verwendet, so wird dieses bevorzugt zentral angeordnet und ist ferner vorzugsweise nicht stab- oder röhrenförmig, sondern vorzugsweise oval, dreieckig, viereckig, mehreckig, stadionbahnförmig, plankonvex oder sternförmig ausgestaltet, um eine Rotation der Teile des Grundkörpers zueinander zu verhindern. Entsprechend ist der andere Teil 1 B des Grundkörpers 1 so ausgestattet, dass er das mindestens eine Führungsmittels 3 des anderen Teils 1A aufnehmen kann. Entlang dieses mindestens einen Führungsmittels 3 können die beiden Teile 1A und 1B des Grundkörpers stufenlos und linear entlang der axialen Achse auseinander bewegt werden.

Als Führungsmittel 3 können beispielsweise die bereits erwähnten Führungsschienen, Führungsstäbe oder Führungsrohre eingesetzt werden, welche dann bevorzugt sind, wenn mindestens zwei Führungsmittel 3A und 3B verwendet werden. Im Grundzustand sind beide Teile 1A und 1B des Grundkörpers 1 ineinandergeschoben. In dieser Position haben auch beide Spreizmittel 2A und 2B den geringsten Abstand voneinander.

An jeweils einen Teil 1A oder 1B des Grundkörpers 1 ist jeweils ein Spreizmittel seitlich angebracht, wodurch durch das stufenlose lineare Auseinanderschieben der beiden Teile 1A und 1B des Grundkörpers 1 auch die beiden Spreizmittel 2A und 2B in gleichem Maße stufenlos linear nur entlang der axialen Achse auseinandergeschoben, d.h. aufgeweitet werden.

Es ist bevorzugt, dass eine Translationsbewegung der beiden Spreizmittel 2A und 2B relativ zueinander nur in einer Dimension, d.h. entlang der Achse durch die Wirbelsäule erfolgen kann.

Das Spreizmittel 2A ist seitlich an dem einen Teil 1A des Grundkörpers 1 angebracht. Vorzugsweise ist die Anbringung derart, dass das Spreizmittel 2A eine Rotationsbewegung um eine senkrecht zur axialen Achse verlaufende Achse ausführen kann. Die Rotationsachse, auf der sich das Spreizmittel 2A befindet verläuft somit senkrecht zur Achse, entlang derer der Grundkörper expandiert werden kann. Die Spreizmittel 2A / 2B sind rotationsflexibel, vorzugsweise bis zu 20° gelagert, um sich den anatomischen Gegebenheiten anpassen zu können: Dies ist wichtig, um Lastspitzen und den daraus folgenden Knochenabbau im Bereich der Kontaktzonen zwischen Knochen und Implantat zu vermeiden.

Das Spreizmittel 2B ist seitlich an dem anderen Teil 1 B des Grundkörpers angebracht und befindet sich vorzugsweise auf derselben Seite des Grundkörpers 1 wie das Spreizmittel 1A. Ferner ist auch bei Spreizmittel 2B vorteilhaft, wenn Spreizmittel 2B auf einer Rotationsachse gelagert ist, welche senkrecht zur axialen Achse verläuft. Zudem ist bevorzugt, wenn die Rotationsachse durch das Spreizmittel 2B parallel zur Rotationsachse durch das Spreizmittel 2A verläuft.

Die mindestens zwei Spreizmittel 2A und 2B können eine beliebige Form besitzen. Vorzugsweise sind die beiden Spreizmittel plankonvex ausgestaltet, d.h. sie haben die Form eines entlang seiner Längsachse geschnittenen Zylinders. Im Grundzustand der Vorrichtung, d.h. im nicht expandierten oder nicht aufgeweiteten oder nicht ausgezogenen Zustand des Grundkörpers, liegen die beiden planen Flächen der beiden Spreizmittel 2A und 2B flach aneinander, so dass beide Spreizmittel zusammen eine Zylinderform bzw. elipsoide Form ergeben.

Insbesondere bevorzugt ist, wenn die mindestens zwei Spreizmittel 2A und 2B nicht deformierbar sind. Auch insbesondere bevorzugt ist, wenn die gesamte Vorrichtung nicht deformierbar oder verformbar oder elastisch ist. Dies bedeutet, dass die Vorrichtung, insbesondere der Grundkörper 1 und die beiden Spreizmittel 2A und 2B aus einem harten Material hergestellt werden, wie beispielsweise medizinischer Edelstahl, Titan oder Titanlegierungen, Tantal, Chrom, Cobalt-Chrom-Legierungen, Vanadium, Wolfram, Molybdän, Kunststoffe wie beispielsweise PEEK (Polyetheretherketon) sowie faserverstärkte Kunststoffe, welche durch den durch die Dornfortsätze erzeugten Druck nur unwesentlich deformiert werden.

Durch diese harten Materialien wird gewährleistet, dass das Ausmaß der Aufweitung der Spreizmittel auch dem Ausmaß der Spreizung der Dornfortsätze entspricht. Demgegenüber würde ein elastisches Material durch den bei größerer Aufweitung überproportional ansteigenden Druck der Dornfortsätze stärker deformiert als bei einer geringeren Aufweitung, so dass kein linearer Zusammenhang zwischen dem Abstand der beiden Spreizmittel und dem Abstand der beiden Wirbelfortsätze zueinander bestehen würde. Es ist jedoch möglich, Materialien zum Einsatz kommen zu lassen, welche eine minimale Eigenflexibilität aufweisen, wie beispielsweise PEEK oder UHMWPE, um einen Knochenabbau im Bereich der Kontaktzonen zu vermeiden.

Somit weist der Grundkörper eine definierte Form auf, welche sich abgesehen von der Aufweitung auch während der Implantation nicht verändert. Ferner besteht der Grundkörper nicht aus einem elastischen Material und ist auch nicht befüllbar oder durch im Inneren des Grundkörpers erzeugten Druck expandierbar. Der Grundkörper besteht vorzugsweise aus einem metallischen und/oder nicht polymeren und/oder formfesten und/oder nicht unter Druck expandierbaren Material.

Sind nun aus der ineinandergeschobenen nicht expandierten Grundstellung die mindestens zwei Spreizmittel 2A und 2B oder die mindestens zwei Teile des Grundkörpers 1 auseinander geschoben worden, so muss durch mindestens ein Fixierungsmittel 4 sichergestellt werden, dass der eingestellte Abstand auch dauerhaft beibehalten wird, d.h. die Spreizmittel ihren Abstand zueinander dauerhaft behalten und nicht dem durch die Dornfortsätze ausgeübten Druck nachgeben.

Als Fixierungsmittel können Stifte, Bolzen, Haken, Stäbe oder Schrauben eingesetzt werden, wobei Gewindestifte und Gewindeschrauben bevorzugt sind. Bei den Ausführungsformen mit einteiligem Grundkörper und zwei auf Führungsmitteln befestigten Spreizmitteln sind mindestens zwei Fixierungsmittel 4A und 4B erforderlich. Bei den Ausführungsformen mit einteiligem Grundkörper und einem festen Spreizmittel und einem auf einem Führungsmittel befestigtem Spreizmittel oder einem zweiteiligen Grundkörper genügt ein Fixierungsmittel 4.

Ferner ist bevorzugt, wenn das mindestens eine Fixierungsmittel 4 eine kegelförmig zulaufende Spitze 5 aufweist. Diese kegelförmig zulaufende Spitze hat ferner vorzugsweise einen Winkel von 45 Grad. Die Spitze 5 ist zentriert angeordnet.

Insbesondere ist bevorzugt, wenn dieses mindestens eine Fixierungsmittel 4 nicht nur zur Fixierung der Spreizmittel 2A und 2B im ausgezogenen Zustand dient, sondern über das mindestens eine Fixierungsmittel 4 auch gleichzeitig der Abstand der beiden Spreizmittel 2A und 2B zueinander eingestellt werden kann.

Dies kann beispielsweise dadurch erreicht werden, dass ein Führungsmittel 3 eine abgeschrägte Fläche 7 aufweist, welche an der Spitze 5 des mindestens einen Fixierungsmittels 4 anliegt. Durch eine Höhenverstellung des mindestens einen Fixierungsmittels 4 gleitet die kegelförmige Spitze 5 entlang der abgeschrägten Fläche 7 eines Führungsmittels 3 in Richtung des spitz zulaufenden Endes dieser gewinkelten Fläche 7, wodurch die Translationsbewegung des Fixierungsmittels 4 entlang der Achse durch das Fixierungsmittel 4 eine Translationsbewegung des Führungsmittels 3 entlang der Achse durch das Führungsmittel 3 erzeugt und gleichzeitig die Position des Führungsmittels 3 fixiert. Vorzugsweise weist die keilförmig zulaufende Fläche 7 denselben Winkel auf, mit dem die Spitze 5 des Fixierungsmittels 4 kegelförmig zulaufend ist. Insbesondere ist bevorzugt, wenn diese beiden Winkel 45 Grad betragen.

Werden Ausführungsformen mit zwei Führungsmitteln 3A und 3B mit jeweils einem angebrachten Spreizmittel 2 und einem vorzugsweise einteiligen Grundkörper 1 bereitgestellt, so ist es bevorzugt, zwei Fixierungsmittel 4A und 4B zum Bewegen und Fixieren der beiden Führungsmittel 3A und 3B einzusetzen, also ein Fixierungsmittel 4 pro Führungsmittel 3.

Bei einer weiteren bevorzugten Ausführungsform der vorliegenden Erfindung wird ein einteiliger Grundkörper 1 verwendet, woran ein Spreizmittel 2A translationsstabil befestigt ist und nur Rotationsbewegungen ausführen kann. Die Rotationsbewegungen können dabei nur um die Längsachse des Spreizmittels 2A ausgeführt werden. Translationsbewegungen des Spreizmittels 2A relativ zum Grundkörper sind nicht möglich. Ferner befindet sich in dem Grundkörper eine Aussparung zur Ausnahme mindestens eines Führungsmittels 3 oder 3A und 3B oder 3A, 3B und 3C, wobei das zweite Spreizmittel 2B an dem mindestens einen Führungsmittel so befestigt ist, dass es Rotationsbewegungen um seine Längsachse, jedoch keine Translationsbewegungen relativ zum mindestens einen Führungsmittel ausführen kann. Die Spreizmittel 2A und 2B können nun durch Verschieben des mindestens einen Führungsmittels entlang der axialen Achse, d.h. entlang der Längsachse der Wirbelsäule in der dafür vorgesehenen Aussparung im Grundkörper 1 translatorisch auseinanderbewegt werden, wodurch eine Aufspreizung der an die Spreizmittel anliegenden Dornfortsätze zweier benachbarter Wirbel erfolgt. Wird die gewünschte Aufspreizung erreicht, so wird das Implantat in seiner aufgeweiteten Position durch mindestens ein Fixierungsmittel fixiert.

Vorteilhaft ist, dass bei allen hierin beschriebenen Ausführungsformen des erfindungsgemäßen Domfortsatzspreizers die fixierte Aufweitung dauerhaft erhalten bleibt, da keine elastischen Materialien verwendet werden, welche nach einer gewissen Zeit der Belastung nachgeben und die gewünschte Aufspreizung der Dornfortsätze sich wieder verringert.

Andere erfindungsgemäße Ausführungsformen verwenden bevorzugt zweiteilige Grundkörper, welche entlang mindestens eines Führungsmittels in Richtung der axialen Achse und vorzugsweise nur in Richtung der axialen Achse distrahiert werden können. Jeweils ein nicht-deformierbares Spreizmittel ist an der Seitenfläche in Richtung der lateralen Achse am jeweiligen Teil des Grundkörpers angebracht. Bei diesen erfindungsgemäßen Ausführungsformen erfolgt die Distraktion nicht mittels einer implantateigenen Vorrichtung wie dem Fixierungsmittel 4 mit kegelförmiger Spitze 5, sondern über ein externes Distraktionsmittel, welche nicht mit implantiert und nur zeitweise während der Operation verwendet wird.

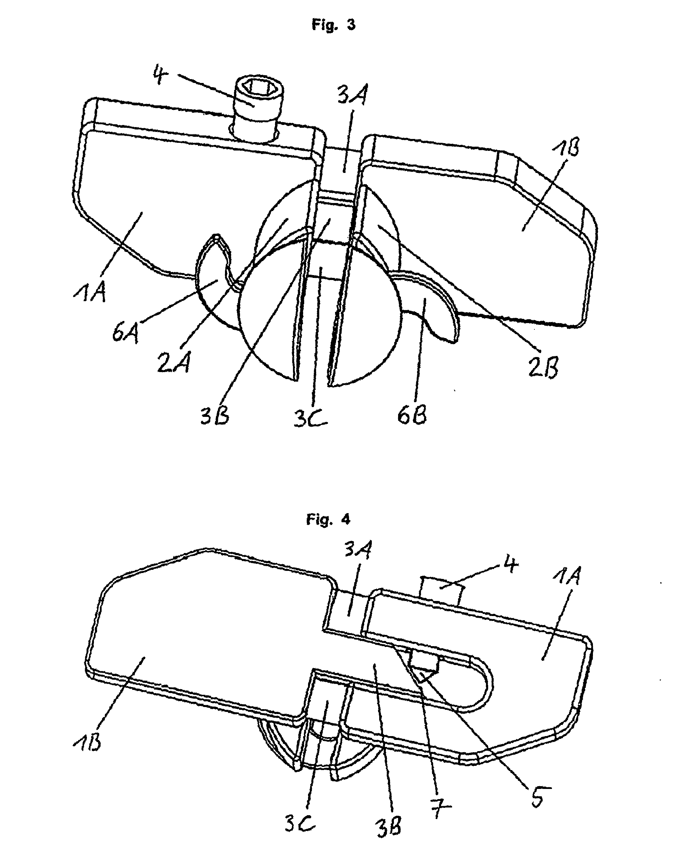

Ein derartiges Distraktionsmittel ist z.B. eine Distraktionszange, welche in jeweils einer Aussparung an einem Teil des Grundkörpers 1A und 1B angesetzt wird. Mittels einer solchen Distraktionszange erfolgt die Aufweitung des Implantats in axialer Richtung vorzugsweise linear und stufenlos. Nach Erreichung der gewünschten Distraktion erfolgt die Fixierung beider Teile des Grundkörpers 1A und 1B relativ zueinander durch mindestens ein Fixierungsmittel. Bei diesem Fixierungsmittel 4 kann es sich um eine Gewindeschraube mit flacher oder unebener oder abgeschrägter Spitze handeln, welche wie in Fig. 4 gezeigt, eingeschraubt wird, bis sie fest an einer entsprechend schrägen Fläche eines Führungsmittels anliegt oder sich durch Druck in ein Führungsmittel eindrückt, um den Grundkörper dadurch zu fixieren. Distraktion und Fixierung des Grundkörpers können somit in-situ erfolgen. Es findet kein vorheriger Schritt des Messens der Implantatgröße statt. Die Distraktionszange dient der Aufnahme des Implantates, der stufenlosen Distraktion der Dornfortsätze und somit in einem Schritt dem Setzen des Implantates.

Das Implantat behält nach Fixierung seine distrahierte Form bei, ohne dem Druck der Dornfortsätze nachzugeben und ohne die einmal eingestellte und fixierte Distraktionstrecke zu verändern.

Der Grundkörper 1A, 1B liegt seitlich der Domfortsätze. Zudem weisen die erfindungsgemäßen Implantate eine abgeschrägte Ecke (direkt unterhalb von 2A bzw. 2B) auf, damit mehr als zwei Level gleichzeitig versorgt werden und die Implantate übereinander Platz finden, wenn mehrere Implantate übereinander verwendet werden, d.h. an angrenzenden Wirbelpaaren auch Implantate eingesetzt werden. Die hintere Kurvation des Grundkörpers dient der besseren Anschmiegung an die Anatomie.

Die Spreizmittel dienen als Auflagefläche für die Dornfortsätze und können vorzugsweise um +/- 20° rotiert werden, um auftretende Lastspitzen abzufangen und eine möglichst großflächige Auflagefläche (Kontaktfläche zw. Anatomie und Implantat) zu gewährleisten. Somit sind bei allen erfindungsgemäßen Ausführungsformen die Spreizmittel rotationsbeweglich um ihre eigene Längsachse am Grundkörper angebracht. Ferner haben die Spreizmittel eine runde, ovale oder plankonvexe Form, um für den Dornfortsatz eine größtmögliche Auflagefläche zu bieten.

Damit nun das Implantat stabil liegt, werden die Dornfortsätze zwischen einem Haltemittel 6A, 6B und dem jeweiligen Teil des Grundkörpers 1A bzw. 1B eingefasst. Dabei ist die Verwendung eines Federelements bevorzugt, so dass das jeweilige Haltemittel automatisch einrastet, wenn es aufgespreizt wird. Dadurch wird die Position der Haltemittel 6A als auch 6B gesichert, um eine Dislokation des Implantates zu verhindern. Die Haltemittel 6A und 6B lagern sich seitlich an der Gegenseite des Grundkörpers an den Domfortsätzen an und verhindern die Dislokation des Implantates.

Das Ausklappen der Haltemittel kann von der dem Implantat gegenüber liegenden Seite der Wirbelsäule erfolgen, was jedoch einen weiteren chirurgischen Eingriff bedeutet. Daher erfolgt das Ausklappen beider Haltemittel vorzugsweise von der gegenüber liegenden Seite des Grundkörpers. Dazu befindet sich jeweils eine Bohrung im Grundkörper oder eine Aussparung am Grundkörper, durch welche ein entsprechendes Instrument bis zum Haltemittel vorgeschoben werden kann, um dieses dann manuell oder über einen Federmechanismus, der durch Druck ausgelöst werden kann, auszuklappen.

Dadurch wird ein unilaterales Einbringen (nur eine Seite der Wirbelsäule wird frei präpariert) des erfindungsgemäßen Implantats ermöglicht, was wiederum einen kleineren Eingriff und ein geringeres Trauma für den Patienten bedeutet und zu kürzeren OP-Zeiten führt.

Bei allen hierin beschriebenen Ausführungsformen liegt das mindestens eine Führungsmittel im wesentlichen weitgehend parallel zur durch die Wirbelsäule verlaufenden axialen Achse und die beiden Spreizmittel 2A und 2B sind betrachtet entlang der Längsachse durch die Spreizmittel senkrecht zum Führungsmittel angeordnet. Ferner ist bevorzugt, dass die Spreizmittel eine Rotation um ihre Längsachse ausführen können, um sich dem aufliegenden Dornfortsatz optimal anpassen zu können. Zudem ist bevorzugt, wenn das mindestens eine Fixierungsmittel entlang seiner Längsachse senkrecht zum Führungsmittel angeordnet ist, wobei ferner zudem eine Anordnung senkrecht zur Längsachse des Spreizmittels bevorzugt ist.

Die erfindungsgemäße Vorrichtung zum Spreizen der Dornfortsätze zweier aufeinanderfolgender Wirbel umfasst also einen Grundkörper 1 mit zwei Spreizmitteln 2A und 2B, wobei sich die Längsachse des Grundkörpers entlang der Wirbelsäule erstreckt. Entspricht der Grundkörper dem Führungsmittel oder ist das mindestens eine Führungsmittel im Grundkörper angeordnet, so liegt auch dieses parallel zur Achse durch die Wirbelsäule. Die beiden Spreizmittel 2A und 2B sind senkrecht zur Achse durch die Wirbelsäule angeordnet und distrahieren die Dornfortsätze entlang der Achse durch die Wirbelsäule. Zum festen Einfassen der Dornfortsätze weist jedes Spreizmittel am zum Grundkörper gegenüberliegenden Ende ein Haltemittel 6A bzw. 6B auf. Spreizmittel und Haltemittel sind rotationsbeweglich um die Längsachse durch das jeweilige Spreizmittel gelagert. Ferner sorgt mindestens ein Fixierungsmittel für die Fixierung des Implantats in seiner distrahierten Position oder kann neben der Fixierung auch selbst die Distraktion stufenlos bewirken. Die Fixierung ist dauerhaft und die Distraktion bzw. die Distraktionsstrecke wird durch die einwirkende Kraft der Dornfortsätze nicht geändert, sondern kann höchstens durch den Operateur verändert werden. Als Distraktion bzw. Distraktionsstrecke wird die Strecke bezeichnet, um welche das Implantat distrahiert, d.h. aufgeweitet wird.

Weitere bevorzugte Ausführungsformen der vorliegenden Erfindung weisen ferner mindestens ein Haltemittel 6 und vorzugsweise ein Haltemittel 6 pro Spreizmittel 2 auf. Vorzugsweise ist das Haltemittel 6A an der dem Grundkörper abgewandten Seite des Spreizmittels 2A und das Haltemittel 6B an der dem Grundkörper abgewandten Seite des Spreizmittels 2B befestigt.

Die vorzugsweise zwei Haltemittel 6A und 6B sind sichelförmig oder halbmondförmig ausgestaltet, flach und an einem Ende drehbar um eine durch das jeweilige Spreizmittel verlaufende Achse angebracht. Durch eine derartige Anbringung können die Haltemittel 6 in einem eingeklappten Grundzustand und einem ausgeklappten Zustand vorliegen. Im eingeklappten Grundzustand liegen beide Haltemittel 6A und 6B wie zwei Sicheln ineinander, wobei der Drehpunkt des Haltemittels 6A den Mittelpunkt des Haltemittels 6B und der Drehpunkt des Haltemittels 6B den Mittelpunkt des Haltemittels 6A bildet.

Wird der Grundkörper nun aufgeweitet bzw. werden die Spreizmittel nun auseinander bewegt, so führt dies zumindest zu einem teilweisen Ausklappen beider Haltemittel 6A und 6B. Das mindestens eine Haltemittel 6 dient dazu, die Vorrichtung nach der Implantation zwischen den aufliegenden Domfortsätzen zu fixieren, damit ein ungewolltes Abgleiten, Weggleiten, Verrutschen oder Verschieben der Vorrichtung zwischen den Wirbeln verhindert wird.

Neben dieser Möglichkeit des Ausklappens der vorzugsweise zwei Haltemittel 6A und 6B können natürlich auch andere Weisen realisiert werden. Eine weitere Möglichkeit besteht darin, ein Haltemittel über einen Federmechanismus auszuklappen, der durch Berührung eines gewissen Punktes am Haltemittel oder Spreizmittel ausgelöst wird. Bevorzugt ist ferner das Ausklappen der Haltemittel durch ein externes Gerät, welches vorzugsweise durch eine entsprechende Bohrung im Grundkörper von der den Spreizmitteln gegenüberliegenden Seite eingeführt und bis zu dem jeweiligen Haltemittel vorgeschoben wird. Diese Bohrung kann dann nicht nur durch den Grundkörper verlaufen, sondern auch im Spreizmittel fortgesetzt werden. Ferner ist bevorzugt, wenn der Ausklappvorgang für die Haltemittel reversibel ist, damit eine eventuelle Reimplantation problemlos durchgeführt werden kann.

Zum leichteren Einfädeln in das Foramen interspinosus kann sich auf oder über den Haltemitteln 6A und 6B eine spitz zulaufende Abdeckung 8A bzw. 8B befinden. In Fig. 3 sind anschaulich diese beiden Abdeckungen zu erkennen, welche näherungsweise die Gestalt eines Viertels einer Kugel aufweisen. Diese Ausgestaltung ist jedoch nicht zwingend. Vorteilhaft sind aber Abdeckungen 8A und 8B, welche in gewisser Weise spitz zulaufen sollten. Ferner ist bevorzugt, wenn beide Abdeckungen 8A sowie 8B einen ähnlichen Umfang wie die jeweiligen Spreizmittel 2A und 2B haben. Diese Abdeckungen 8A und 8B können zentriert auf den Haltemitteln 6A bzw. 6B angebracht sein, wobei aber eine versetzte Anbringung der Abdeckungen 8A bzw. 8B bevorzugt ist, da diese auch zu einem leichteren Einfädeln in das Foramen interspinosus beträgt. Anstelle einer versetzten oder dezentrierten Anbringung der Abdeckungen 8A bzw. 8B können auch Abdeckungen mit dezentraler bzw. versetzter Spitze verwendet werden. Beide Abdeckungen 8A und 8B sind vorzugsweise derart gelagert, dass eine Rotation um die durch das jeweilige Spreizmittel 2 verlaufende Achse möglich ist.

Ferner ist bevorzugt, wenn die einzelnen Komponenten der erfindungsgemäßen Vorrichtung mit einer keramischen Beschichtung überzogen sind. Keramische Beschichtungen umfassen Nitride, Carbide und Phosphide von bevorzugt Halbmetallen und Metallen bzw. Metalllegierungen. Beispiele für keramische Beschichtungen sind Bornitride, Titan-Niob-Nitrid, Titan-Calcium-Phosphid (Ti-Ca-P), Cr-Al-N, Ti-Al-N, Cr-N, TiAIN-CrN, Ti-Al-C, Cr-C, TiAlC-CrC, Zr-Hf-N, Ti-Hf-C-N, Si-C-N-Ti, Si-C-N sowie DLC (Diamond Like Carbon).

### Abkürzungsverzeichnis:

- 1: Grundkörper
- 1A: Teil A des Grundkörpers
- 1B: Teil B des Grundkörpers
- 2: Spreizmittel
- 2A: Spreizmittel A
- 2B: Spreizmittel B
- 3: Führungsmittel
- 3A: Führungsmittel A
- 3B: Führungsmittel B
- 3C: Führungsmittel C
- 4: Fixierungsmittel
- 4A: Fixierungsmittel A
- 4B: Fixierungsmittel B
- 5: kegelförmige Spitze des Fixierungsmittels
- 6: Haltemittel
- 6A: Haltemittel A
- 6B: Haltemittel B
- 7: an die Spitze des Fixierungsmittels anliegende Fläche
- 8A: Abdeckung A
- 8B: Abdeckung B
- 9A/9B: Eingriffsloch für Distraktionszange
- 10A: Schnappmechanismus für Haltemittel 6A
- 10B: Schnappmechanismus für Haltemittel 6B

### Figurenbeschreibung

- Figur 1: zeigt eine Seitenansicht einer Ausführungsform der erfindungsgemäßen Vorrichtung im teilweise zusammengeschobenen Grundzustand mit sich ausklappenden sichelförmigen Haltemitteln 6A, 6B;
- Figur 2: zeigt eine Seitenansicht einer Ausführungsform der erfindungsgemäßen Vorrichtung mit zweiteiligem Grundkörper 1 im zusammengeschobenen Grundzustand und den beiden mit ihren planen Flächen aneinanderliegenden Spreizmitteln 2A, 2B und den beiden sichelförmigen Haltemitteln 6A, 6B im eingeklappten Zustand;
- Figur 3: zeigt eine Seitenansicht einer Ausführungsform der erfindungsgemäßen Vorrichtung entlang einer durch die Spreizmittel 2 verlaufenden Achse mit zweiteiligem Grundkörper 1A, 1B im expandierten Zustand und mit eindrehbarem Fixierungsmittel 4, aufgeweiteten Spreizmitteln 2A, 2B und ausgeklappten Haltemitteln 6A, 6B;
- Figur 4: zeigt eine Seitenansicht einer Ausführungsform der erfindungsgemäßen Vorrichtung von der den Spreizmitteln 2 gegenüberliegenden Seite. Ein zweiteiliger Grundkörper 1A, 1B im teilweise aufgeweiteten Zustand und teilweise eingeschraubtem Fixierungsmittel 4 ist zu erkennen, wobei die kegelförmige Spitze 5 des Fixierungsmittels 4 an der entsprechend abgeschrägten Fläche 7 des nicht mit dem Fixierungsmittel 4 versehenen Teil des Grundkörpers 1 anliegt;
- Figur 5: zeigt die aufgeweitete Form einer weiteren Ausführungsform der erfindungsgemäßen Vorrichtung mit einem zweiteiligen Grundkörper 1A, 1 B und einem stadionbahnförmigen Führungsmittel 3, den beiden Spreizmitteln 2A und 2B sowie den beiden Haltemitteln 6A und 6B, welche beispielsweise über einen Schnappmechanismus 10A bzw. 10B ausgeklappt werden können;
- Figur 6: zeigt eine Seitenansicht des Implantats gemäß Fig. 5 im nicht distrahierten Zustand;
- Figur 7: zeigt eine weitere Seitenansicht des Implantats gemäß Fig. 5 im komprimierten Zustand;
- Figur 8: zeigt eine schematische Ansicht einer weiteren Ausführungsform der erfindungsgemäßen Vorrichtung in der einfachsten Variante;
- Figur 9: zeigt eine weitere perspektivische Ansicht der Ausführungsform gemäß Fig. 8, wobei ein stabförmiges Führungsmittel 3 sowie zwei entlang dem Führungsmittel beweglich gelagerte Spreizmittel schematisch gezeigt sind;
- Figur 10: zeigt eine weitere Ansicht der Ausführungsform gemäß Fig. 8 mit Blick in ventrale Richtung (d.h. in Richtung Bauch);
- Figur 11: zeigt eine weitere Ansicht der Ausführungsform gemäß Fig. 8 in laterale Richtung.

### Ausführungsbeispiele

Bevorzugte Ausführungsformen der erfindungsgemäßen Vorrichtung werden nun anhand der Beispiele diskutiert, wobei zu berücksichtigen ist, dass die diskutierten Beispiele vorteilhafte Ausgestaltungen der Erfindung wiedergeben, jedoch den Schutzumfang nicht auf diese Ausführungsformen beschränken.

### Beispiel 1

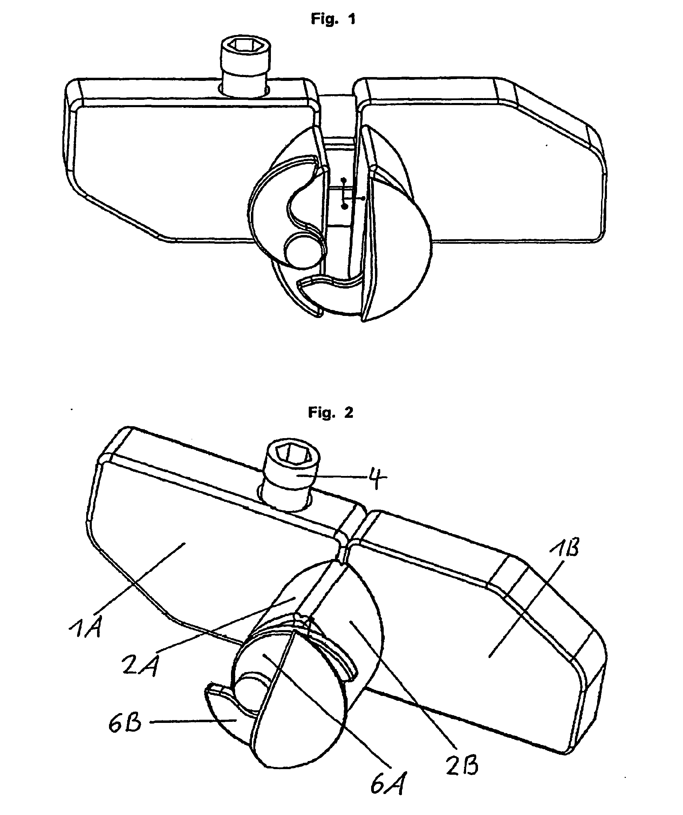

Figur 1 zeigt eine bevorzugte Ausführungsform, deren Einzelkomponenten aus Titan gefertigt sind und einen zweiteiligen Grundkörper 1A und 1B, drei Führungsmittel 3A, 3B, 3C, zwei Spreizmittel 2A und 2B, ein Fixierungsmittel 4 sowie zwei Haltemittel 6A und 6B umfassen.

Figur 2 zeigt eine Ausführungsform der erfindungsgemäßen Vorrichtung im nicht expandierten zusammengeschobenen Grundzustand. Die beiden Teile des Grundkörpers 1A und 1B liegen aneinander. Ebenfalls liegen auch die planen Flächen der beiden plankonvex ausgestalteten Spreizmittel 2A und 2B aufeinander, so dass beide Spreizmittel 2A und 2B zusammen einen Zylinder oder einen zusammengedrückten Zylinder ergeben. Das Fixierungsmittel 4 in Form einer Gewindeschraube ist herausgedreht, so dass ein Teil der Gewindeschraube aus dem einen Teil 1A des Grundkörpers 1 herausragt. Die beiden am Kopf der Spreizmittel 2 angebrachten sichelförmigen Haltemittel 6A und 6B sind im eingeklappten Zustand.

Die Vorrichtung wird im Grundzustand befindlich implantiert, d.h. zwischen die beiden Dornfortsätze der zu spreizenden Wirbel gebracht, ohne dass eine Durchtrennung des Ligamentum supraspinosum erforderlich wäre, was einen großen Vorteil der erfindungsgemäßen Implantate darstellt. Nach dem Platzieren zwischen den Domfortsätzen erfolgt die Aufweitung bzw. Expansion der Vorrichtung durch das Einschrauben der Gewindeschraube.

Figur 4 zeigt eine Ansicht des zweiteiligen Grundkörpers 1 von der den beiden Spreizmitteln 2 gegenüberliegenden Seite. Es sind drei Führungsmittel vorhanden, von denen zwei als zylinderförmige Stifte ausgestaltet sind. Die beiden zylinderförmigen Stifte 3A und 3C sind entweder am Teil 1A oder am Teil 1B des Grundkörpers befestigt oder Teil des Grundkörpers selbst oder ein Führungsmittel 3A oder 3C ist am Teil 1A und das andere Führungsmittel am Teil 1B angebracht bzw. zu dem jeweiligen Teil 1A oder 1B gehörig. Der andere Teil des Grundkörpers weist entsprechende Bohrungen oder Aussparungen zur Aufnahme der Führungsmittel 3A und 3C auf.

Der nicht mit dem Fixierungsmittel 4 versehene Teil 1 B des Grundkörpers 1 weist ein Führungsmittel 3B auf, welches an seinem freien Ende in einer schrägen Fläche 7 ausläuft, welche an der kegelförmigen Spitze 5 des Fixierungsmittels 4 anliegt.

Das als Gewindeschraube ausgestaltete Fixierungsmittel 4 kann in den Teil 1A des Grundkörpers 1 entlang einer Achse, welche senkrecht zur axialen Achsen, d.h. der Achse entlang der Wirbelsäule und senkrecht zu einer durch ein Spreizmittel 2 verlaufenden Achse verläuft, hineingedreht werden.

Das Fixierungsmittel 4 weist ein spitz zulaufendes Ende 5 auf. Das kegelförmige Ende 5 hat vorzugsweise einen Winkel von 45°. Die gewinkelte Fläche 7 des Führungsmittels 3B weist ebenfalls vorzugsweise einen Winkel von 45° auf, so dass sich Fläche 7 und Spitze 5 entlang einer Geraden berühren.

Wird nun das Fixierungsmittel entlang seiner Mittelachse in den Teil 1A des Grundkörpers 1 gedreht, so verschiebt sich die Gerade, in der sich Fläche 7 und Spitze 5 berühren in Richtung der Kante von Fläche 7, wodurch eine Translationsbewegung beider Teile 1A und 1B des Grundkörpers 1 entlang der axialen Achse auseinander initiiert wird. Zugleich verhindert das Fixierungsmittel 4, dass durch den durch beide Dornfortsätze erzeugten Druck beide Teile 1A und 1 B des Grundkörpers 1 wieder ineinander geschoben werden. Die Führungsmittel 3A und 3C gewährleisten, dass die Translationsbewegung der beiden Teile 1A und 1B des Grundkörpers 1 nur in axialer Richtung erfolgen kann und nicht zusätzlich von einer Rotationsbewegung der beiden Teile 1A und 1B umeinander begleitet wird.

Bei maximaler Expansion des Grundkörpers 1 ist das Fixierungsmittel 4 vollständig in den Teil 1A des Grundkörpers hineingedreht und die äußere Spitze der kegelförmigen Spitze 5 ist an der Außenkante der schrägen Fläche 7 angelangt. Die beiden Teile 1A und 1B des Grundkörpers 1 sind nun maximal aufgeweitet und die Aufweitung beträgt 20 mm.

Eine Vorderansicht der Vorrichtung im expandierten Zustand bietet Figur 3. Die beiden Teile 1A und 1B des Grundkörpers 1 sind entlang der Achsen durch die beiden Führungsmittel 3A und 3C auseinandergeschoben. Den Abstand, den nun die beiden Teile 1A und 1B des Grundkörpers zueinander aufweisen, haben nun auch die beiden Spreizmittel 2A und 2B zueinander. Das Fixierungsmittel 4 ist bis auf den Schraubenkopf im Teil 1A des Grundkörpers versenkt. Die beiden Haltemittel 6A und 6B an den Außenflächen der Spreizmittel 2A und 2B sind ausgeklappt und sichern eine feste Lage der Vorrichtung zwischen den Wirbelkörpern, da sie den jeweiligen Domfortsatz, der auf dem jeweiligen Spreizmittel aufliegt, zwischen sich und dem jeweiligen Teil des Grundkörpers 1 einklemmen. Somit wird der auf dem Spreizmittel 2B aufliegende Dornfortsatz durch das Haltemittel 6B zwischen dem Haltemittel 6B und dem Teil 1B des Grundkörpers 1 fixiert.

### Beispiel 2

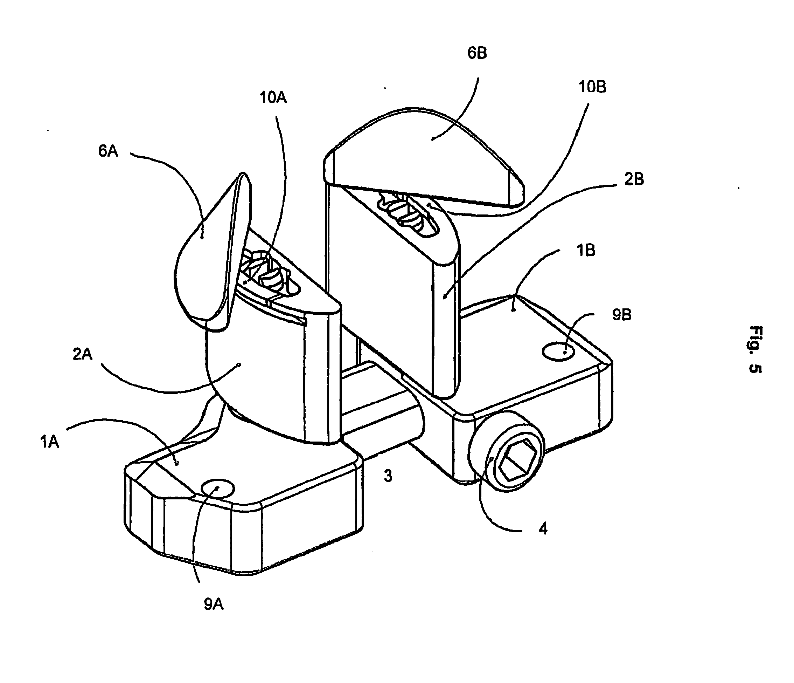

Figur 5 zeigt eine weitere bevorzugte Ausführungsform der vorliegenden Erfindung, deren Einzelkomponenten aus Titan gefertigt sind und einen zweiteiligen Grundkörper 1A und 1B, ein Führungsmittel 3, zwei Spreizmittel 2A und 2B, ein Fixierungsmittel 4 sowie zwei Haltemittel 6A und 6B umfassen.

Jeder Teil des Grundkörpers 1A und 1B weist eine Aussparung 9A bzw. 9B zur Aufnahme einer Distraktionszange auf.

Fig. 5 zeigt den Grundkörper im distrahierten Zustand. Das eine Führungsmittel 3 ist kreisbahnförmig so wie eine Laufbahn im Stadion ausgestaltet, damit keine Rotation der beiden Teile des Grundkörper 1A und 1B relativ zueinander möglich sind. Das Führungsmittel 3 ist fest mit dem Teil 1A des Grundkörpers verbunden und nicht beweglich relativ zum Teil 1A oder es bildet mit dem Teil 1A des Grundkörpers eine Einheit. Der Teil 1 B des Grundkörpers weist eine entsprechende Aussparung zur translationsbeweglichen Aufnahme des Führungsmittels auf, so dass beide Teile des Grundkörpers durch eine Gleitbewegung des Führungsmittels in der Aussparung im Teil 1 B stufenlos linear entlang der axialen Achse aufgeweitet bzw. distrahiert werden können.

Ist die gewünschte Distraktion erreicht, so werden die beiden Teile des Grundkörpers 1A und 1B zueinander durch das Fixierungsmittel 4 hier in Form einer Gewindeschraube fixiert. Durch Druck auf das Fixierungsmittel wird mittels des Fixierungsmittels der Distraktionsweg fixiert.

Die beiden Haltemittel 6A und 6B wurden über den jeweiligen Schnappmechanismus 10A bzw. 10B ausgeklappt und klemmen den jeweiligen Dornfortsatz zwischen sich und dem gegenüberliegenden Teil 1A bzw. 1B des Grundkörpers ein.

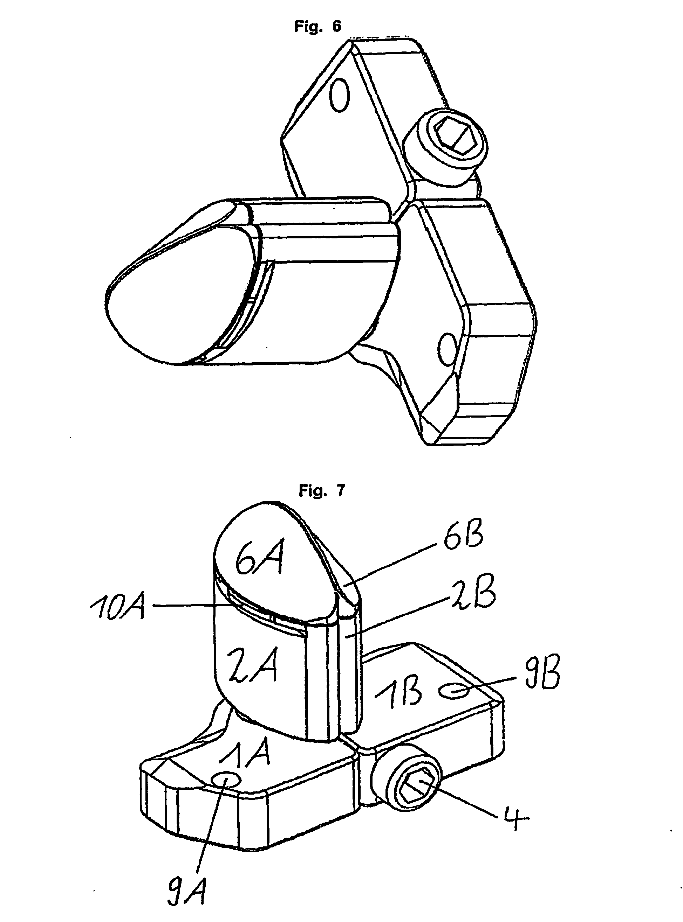

Die erfindungsgemäße Vorrichtung wird im Grundzustand befindlich so wie in Fig. 6 gezeigt implantiert, d.h. zwischen die beiden Dornfortsätze der zu spreizenden Wirbel gebracht, ohne dass eine Durchtrennung des Ligamentum supraspinosum erforderlich wäre, was einen großen Vorteil der erfindungsgemäßen Implantate darstellt. Der Grundkörper liegt dabei seitlich der Wirbelsäule, d.h. er liegt in der durch die axiale Achse und die ventral-dorsale Achse aufgespannte Ebene. J Nach dem Platzieren zwischen den Dornfortsätzen erfolgt die Aufweitung bzw. Distraktion der Vorrichtung durch eine externe Distraktionszange.

Die Distraktionszange wird dazu in die Aussparungen 9A und 9B eingesetzt und das Implantat mittels dieser Distraktionszange stufenlos auf die gewünschte Aufweitung gebracht. Das Fixierungsmittel in Form einer Gewindeschraube wird angezogen, bis durch entsprechenden Druck auf das Führungsmittel eine Fixierung der distrahierten Teile 1A und 1B des Grundkörpers zueinander erreicht wird.

Mittels eines weiteren externen Werkzeugs werden nun die Haltemittel durch direkten Kontakt ausgeklappt, um das gesamte Implantat zu fixieren oder durch einen implantateigenen Klappmechanismus ausgeklappt, der mittels eines externen Werkzeugs ausgelöst wird. Diese externe Werkzeug wird von der entgegengesetzten Seite des Grundkörpers durch eine entsprechende Führung im Grundkörper durch diesen hindurch eingeführt, um den Klappmechanismus auszulösen. Diese Ausführungsform hat den weiteren Vorteil, dass bei der Implantation nur eine Seite der Wirbelsäule freigelegt werden muss und von dieser Seite das Implantat zwischen die benachbarten Dornfortsätze eingesetzt werden kann und auch die Haltemittel von dieser Seite her ausgeklappt werden können.

### Beispiel 3

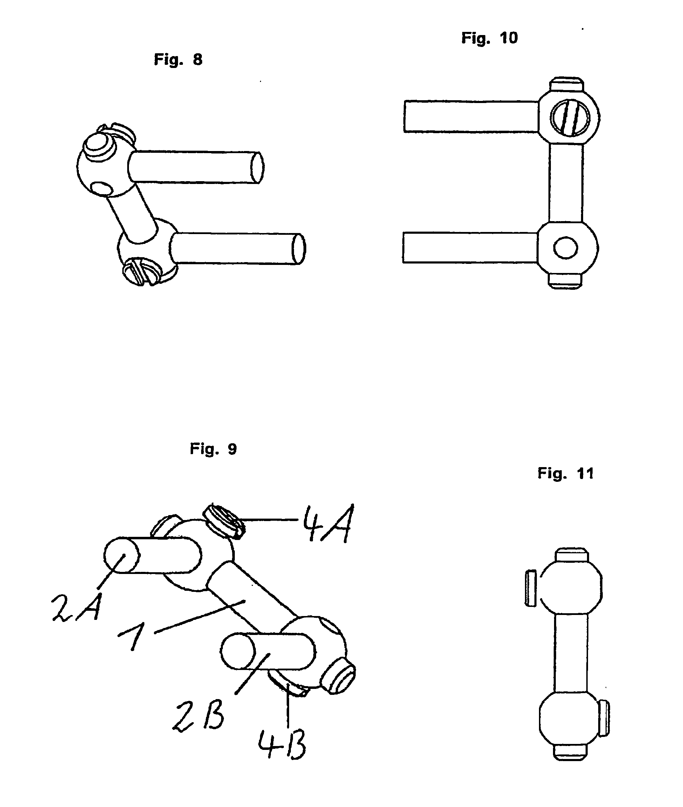

Die Figuren 8 bis 10 zeigen die einfachste Ausführungsform, bei welcher der Grundkörper 1 einteilig ausgestaltet ist und zwei Spreizmittel 2A und 2B entlang des Grundkörpers 1 und entlang der axialen Achse stufenlos mittels einer externen Distraktionszange aufgeweitet werden können.

Beide Spreizmittel weisen ein Fixierungsmittel 4A und 4B auf, um die Spreizmittel relativ zueinander zu fixieren. Die beiden Fixierungsmittel sind in Form einer Gewindeschraube ausgestaltet. Die Spreizmittel 2A und 2B sind nur schematisch in Rohrform in den Figuren 8 - 10 angedeutet und die Haltemittel sind nicht gezeigt.

## Patentansprüche

1. Vorrichtung zum Spreizen der Dornfortsätze zweier aufeinanderfolgender Wirbel, umfassend einen Grundkörper (1) mit zwei Spreizmitteln (2A, 2B), wobei die beiden Spreizmittel (2A, 2B) nur entlang der axialen Achse durch die Wirbelsäule translationsbeweglich stufenlos aufgeweitet und **dadurch gekennzeichnet, dass** die beiden spreizmittel (2A, 2B) mittels mindestens eines Fixierungsmittels (4) fixiert werden können, wobei es sich bei dem mindestens einen Fixierungmittel (4) um einen Gewindestift oder eine Gewindeschraube handelt.

2. Vorrichtung gemäß Anspruch 1, **dadurch gekennzeichnet, dass** der Grundkörper (1) formstabil und nicht verformbar oder deformierbar ist.

3. Vorrichtung gemäß Anspruch 1 oder 2, **dadurch gekennzeichnet, dass** der Grundkörper (1) zweiteilig aufgebaut ist.

4. Vorrichtung gemäß Anspruch 3, **dadurch gekennzeichnet, dass** die Spreizmittel formstabil und nicht deformierbar sind.

5. Vorrichtung gemäß eines der vorhergehenden Ansprüche, **dadurch gekennzeichnet, dass** der zweiteilige Grundkörper (1) aus zwei ineinandergreifenden, ausziehbaren, aufweitbaren, ineinander schiebbaren und/oder aneinander entlang gleitbaren Teilen (1A, 1B) besteht.

6. Vorrichtung gemäß eines der vorhergehenden Ansprüche, **dadurch gekennzeichnet, dass** mindestens ein Teil des Grundkörpers (1) mit mindestens einem Führungsmittel (3) ausgestattet ist und der andere Teil des Grundkörpers (1) zur Aufnahme des mindestens einen Führungsmittels (3) ausgestattet ist.

7. Vorrichtung gemäß Anspruch 6, **dadurch gekennzeichnet, dass** der Grundkörper (1) entlang der Längsachse des mindestens einen Führungsmittels (3) stufenlos und linear aufgeweitet werden kann.

8. Vorrichtung gemäß eines der vorhergehenden Ansprüche, **dadurch gekennzeichnet, dass** die Spreizmittel (2A, 2B) entlang der axialen Achse stufenlos und linear auseinander bewegt werden können.

9. Vorrichtung gemäß eines der vorhergehenden Ansprüche, **dadurch gekennzeichnet, dass** an beiden Teilen des Grundkörpers (1A, 1B) jeweils ein Spreizmittel (2A, 2B) seitlich angebracht ist.

10. Vorrichtung gemäß eines der vorhergehenden Ansprüche, **dadurch gekennzeichnet, dass** die Spreizmittel (2A, 2B) plankonvex ausgestaltet sind.

11. Vorrichtung gemäß eines der vorhergehenden Ansprüche, **dadurch gekennzeichnet, dass** die Spreizmittel (2A, 2B) drehbar jeweils um eine senkrecht zur axialen Achse verlaufenden Achse gelagert sind.

12. Vorrichtung gemäß eines der vorhergehenden Ansprüche, **dadurch gekennzeichnet, dass** das mindestens eine Fixierungsmittel (4) eine kegelförmig zulaufende Spitze aufweist.

13. Vorrichtung gemäß eines der vorhergehenden Ansprüche, **dadurch gekennzeichnet, dass** der nicht mit dem Fixierungsmittel (4) versehene Teil des Grundkörpers (1) eine an die kegelförmige Spitze (5) des mindestens einen Fixierungsmittels (4) anliegende Fläche (7) aufweist.

14. Vorrichtung gemäß eines der vorhergehenden Ansprüche, **dadurch gekennzeichnet, dass** der Winkel, mit dem die kegelförmige Spitze (5) des mindestens einen Fixierungsmittels (4) zuläuft, dem Winkel entspricht, mit dem die an die kegelförmige Spitze (5) des mindestens einen Fixierungsmittels (4) anliegende Fläche (7) des einen Teils des Grundkörpern (1) abgeschrägt ist.

15. Vorrichtung gemäß eines der vorhergehenden Ansprüche, **dadurch gekennzeichnet, dass** die Spreizmittel (2A, 2B) an der dem Grundkörper abgewandten Seite mindestens ein Haltemittel (6A, 6B) aufweisen.

16. Vorrichtung gemäß eines der vorhergehenden Ansprüche, **dadurch gekennzeichnet, dass** das mindestens eine Haltemittel (6) pro Spreizmittel (2) ausklappbar gelagert ist.

17. Vorrichtung gemäß eines der vorhergehenden Ansprüche, **dadurch gekennzeichnet, dass** das mindestens eine Haltemittel (6) pro Spreizmittel (2) sichelförmig ausgestaltet ist.

18. Vorrichtung gemäß eines der vorhergehenden Ansprüche, **dadurch gekennzeichnet, dass** das jeweilige Haltemittel (6) drehbar um eine senkrecht zur axialen Achse durch das jeweilige Spreizmittel (2) verlaufenden Achse gelagert ist.

19. Vorrichtung gemäß eines der vorhergehenden Ansprüche, **dadurch gekennzeichnet, dass** die mindestens zwei Haltemittel (6A, 6B) derart gelagert sind, dass sie gleichzeitig mit dem Aufweiten der Spreizmittel (2A, 2B) ausgeklappt werden.

## Claims

1. Device for the distraction of the spinous processes of two adjacent vertebrae, comprising a central body (1) having two distraction means (2A, 2B), wherein the two distraction means (2A, 2B) can be expanded in an infinitely variable manner only along the axial axis running through the spinal column by a translational movement, and **characterized in that** the two distraction means (2A, 2B) can be fixed by at least one fixation means (4), wherein the at least one fixation means (4) is a threaded pin or a threaded screw.

2. Device according to claim 1, **characterized in that** the central body (1) is dimensionally stable and not ductile or deformable.

3. Device according to claim 1 or 2, **characterized in that** the central body (1) is composed of two pieces.

4. Device according to claim 3, **characterized in that** the distraction means are dimensionally stable and not deformable.

5. Device according to one of the preceding claims, **characterized in that** the two-piece central body (1) consists of two engaging pieces (1A, 1 B) which can be expanded, extended, interleaved and/or can slide along each other.

6. Device according to one of the preceding claims, **characterized in that** at least one piece of the central body (1) is provided with at least one guiding means (3) and that the other piece of the central body (1) is designed such that it can receive the at least one guiding means (3).

7. Device according to claim 6, **characterized in that** the central body (1) can be expanded in an infinitely variable manner and in a linear manner along the longitudinal axis of the at least one guiding means (3).

8. Device according to one of the preceding claims, **characterized in that** the distraction means (2A, 2B) can be moved apart in an infinitely variable manner and in a linear manner along the axial axis.

9. Device according to one of the preceding claims, **characterized in that** one distraction means (2A, 2B), respectively, is laterally arranged on both pieces of the central body (1A, 1B).

10. Device according to one of the preceding claims, **characterized in that** the distraction means (2A, 2B) are designed in a piano-convex manner.

11. Device according to one of the preceding claims, **characterized in that** the distraction means (2A, 2B) are respectively arranged such that they can be rotated around an axis which is perpendicular to the axial axis.

12. Device according to one of the preceding claims, **characterized in that** the at least one fixation means (4) has a conically tapered tip.

13. Device according to one of the preceding claims, **characterized in that** the piece of the central body (1) which is not provided with the fixation means (4) has a surface (7) which abuts on the conical tip (5) of the at least one fixation means (4).

14. Device according to one of the preceding claims, **characterized in that** the angle in which the conical tip (5) of the at least one fixation means (4) tapers, corresponds to the angle in which the surface (7) of the one piece of the central body (1) abutting the conical tip (5) of the at least one fixation means (4) is beveled.

15. Device according to one of the preceding claims, **characterized in that** the distraction means (2A, 2B) have at least one holding means (6A, 6B) on the side which is not facing the central body.

16. Device according to one of the preceding claims, **characterized in that** the at least one holding means (6) per distraction means (2) is arranged such that it can be spread out.

17. Device according to one of the preceding claims, **characterized in that** the at least one holding means (6) per distraction means (2) is designed in the form of a sickle.

18. Device according to one of the preceding claims, **characterized in that** the respective holding means (6) is arranged such that it can be rotated around an axis which is perpendicular to the axial axis and which is running through the respective distraction means (2).

19. Device according to one of the preceding claims, **characterized in that** the at least two holding means (6A, 6B) are arranged such that they are spread out simultaneously to the expansion of the distraction means (2A, 2B).

## Revendications

1. Dispositif destiné à écarter les apophyses épineuses de deux vertèbres adjacents, comprenant un corps de base (1) doté de deux moyens d'écartement (2A, 2B), dans lequel les deux moyens d'écartement (2A, 2B) peuvent être dilatés et fixés de manière continue seulement au long de l'axe axial à travers la colonne vertébrale selon un mouvement de translation, et **caractérisé en ce que** les deux moyens d'écartement (2A, 2B) peuvent être fixés par au moins un moyen de fixation (4), dans lequel le au moins un moyen de fixation (4) est une tige filetée ou un vis à filet.

2. Dispositif selon la revendication 1, **caractérisé en ce que** le corps de base (1) est stable concernant la forme et n'est pas déformable ou ductile.

3. Dispositif selon les revendications 1 ou 2, **caractérisé en ce que** le corps de base (1) se compose de deux parties.

4. Dispositif selon la revendication 3, **caractérisé en ce que** les moyens d'écartement sont stables concernant la forme et ne sont pas ductiles.

5. Dispositif selon l'une quelconque des revendications précédentes, **caractérisé en ce que** le corps de base (1) composé de deux parties se compose de deux parties (1A, 1B) dont l'une peut s'engager dans l'autre, qui peuvent être dépliées, qui peuvent être dilatées et dont l'une peut être poussée dans l'autre et/ ou dont l'une peut glisser tout au long de l'autre.

6. Dispositif selon l'une quelconque des revendications précédentes, **caractérisé en ce que** au moins une partie du corps de base (1) est dotée d'au moins un moyen de guidage (3) et que l'autre partie du corps de base (1) est configurée afin de recevoir le au moins un moyen de guidage (3).

7. Dispositif selon la revendication 6, **caractérisé en ce que** le corps de base (1) peut être dilaté de manière continue et linéaire tout au long de l'axe longitudinal du au moins un moyen de guidage (3).

8. Dispositif selon l'une quelconque des revendications précédentes, **caractérisé en ce que** les moyens d'écartement (2A, 2B) peuvent être déplacés de manière continue et linéaire l'un s'éloignant de l'autre tout au long de l'axe axial.

9. Dispositif selon l'une quelconque des revendications précédentes, **caractérisé en ce que** respectivement un moyen d'écartement (2A, 2B) est attaché latéralement aux deux parties du corps de base (1A, 1 B).

10. Dispositif selon l'une quelconque des revendications précédentes, **caractérisé en ce que** les moyens d'écartement (2A, 2B) sont d'une configuration plan-convexe.

11. Dispositif selon l'une quelconque des revendications précédentes, **caractérisé en ce que** les moyens d'écartement (2A, 2B) sont montés de manière qu'ils peuvent pivoter autour d'un axe étant perpendiculaire par rapport à l'axe axial.

12. Dispositif selon l'une quelconque des revendications précédentes, **caractérisé en ce que** le au moins un moyen de fixation (4) dispose d'une pointe se terminant de manière conique.

13. Dispositif selon l'une quelconque des revendications précédentes, **caractérisé en ce que** la partie du corps de base (1) pas dotée d'un moyen de fixation (4) dispose d'une surface (7) adjacente à la pointe (5) conique du au moins un moyen de fixation (4).

14. Dispositif selon l'une quelconque des revendications précédentes, **caractérisé en ce que** l'angle selon lequel se termine la pointe (5) conique du au moins un moyen de fixation (4) correspond à l'angle selon lequel la surface (7) de la partie respective du corps de base (1) adjacente à la pointe (5) conique du au moins un moyen de fixation (4) est inclinée.

15. Dispositif selon l'une quelconque des revendications précédentes, **caractérisé en ce que** les moyens d'écartement (2A, 2B) disposent d'au moins un moyen de soutien (6A, 6B) sur le côté détourné du corps de base.

16. Dispositif selon l'une quelconque des revendications précédentes, **caractérisé en ce que** le au moins un moyen de soutien (6) par moyen d'écartement (2) est monté de manière dépliable.

17. Dispositif selon l'une quelconque des revendications précédentes, **caractérisé en ce que** le au moins un moyen de soutien (6) par moyen d'écartement (2) dispose d'une forme de faucille.

18. Dispositif selon l'une quelconque des revendications précédentes, **caractérisé en ce que** le moyen de soutien (6) respectif est monté de manière qu'il peut pivoter autour d'un axe étant perpendiculaire par rapport à l'axe axial et passant à travers du moyen d'écartement (2) respectif.

19. Dispositif selon l'une quelconque des revendications précédentes, **caractérisé en ce que** les au moins deux moyens de soutien (6A, 6B) sont montés de manière qu'ils peuvent être dépliés en même temps que les moyens d'écartement (2A, 2B) sont dilatés.
